# EUROPÄISCHE PATENTSCHRIFT

(11) **EP 1 095 633 B1**
(45) Veröffentlichungstag und Bekanntmachung des Hinweises auf die Patenterteilung: **29.12.2004**
(21) Anmeldenummer: 00250352.2
(22) Anmeldetag: 26.10.2000
(51) Int. Cl.: A61F 2/06

(54) **Stent mit geschlossener Struktur**
Stent having a closed structure
Stent à structure fermée

(30) Priorität: 26.10.1999 DE 19951611
(43) Veröffentlichungstag der Anmeldung: 02.05.2001
(73) Patentinhaber: BIOTRONIK Mess- und Therapiegeräte GmbH & Co Ingenieurbüro Berlin, 12359 Berlin (DE)
(72) Erfinder: Lootz, Daniel, 18055 Rostock (DE)
(74) Vertreter: Eisenführ, Speiser & Partner

(56) Entgegenhaltungen:
- DE-A- 19 722 857
- DE-A- 19 950 756

## Beschreibung

Die Erfindung betrifft einen Stent mit einem rohrförmigen Abschnitt, der Ausnehmungen aufweist.

Derartige Stents sind aus dem Stand der Technik in vielfältiger Weise bekannt. Diese Stents werden u.a. im Zusammenhang mit der perkutanen transluminalen Angioplastie (PCTA, Percutaneous Transluminal Balloon Angioplasty) in der Gefäßchirurgie des Herzens verwendet. Stents können jedoch auch dazu dienen, andere Körperöffnungen aufzuweiten oder aufgeweitet zu halten. Diesem medizinischen Verfahren geht zunächst die Bestimmung des Ortes der Verengung eines Herzkranzgefäßes voraus. Anschließend wird ein sogenannter Angiopfastiebal-Ion in der Arterie, die die Verengung, die sogenannte Stenose aufweist, an den Ort der Stenose verbracht und dort aufgeblasen. Durch die radial nach außen gerichtete Kraft des aufgeblasenen Ballons wird die Verengung aufgeweitet und im optimalen Fall der ursprüngliche Durchtrittsquerschnitt der zuvor verengten Arterie wieder hergestellt. Neben der erfolgreichen Aufweitung des Gefäßes kann es jedoch zu Nebenwirkungen kommen, die lokale Risse in der Arterie, Zersetzungen und Vorsprünge von Plättchen in das Lumen der Arterie umfassen, so daß es trotz der Aufweitung zu einer Blockade des Gefäßes kommen kann. Darüber hinaus ist es möglich, daß durch elastisches Zurückfedern der Gefäßwand und/oder durch das Wachstum der Intima des Gefäßes erneut eine Stenose auftreten kann. Dies tritt statistisch innerhalb von sechs Monaten bei über 30% der Patienten auf, die mit PCTA behandelt wurden.

Um nun unmittelbar nach der Aufweitung des Gefäßes eine relativ glatte Innenwand des Gefäßes sicherzustellen und eine erneute Stenose vermeiden zu können, wurden die eingangs genannten Stents entwickelt. Diese kleinen Röhrchen dienen u.a. im Zusammenhang mit der PCTA dazu, den durch die Ballonangioplastie erzeugten Gefäßdurchtrittsquerschnitt aufrecht zu erhalten, um somit einen langfristigen Erfolg der PCTA sicherzustellen.

Der Erfolg dieses sogenannten Stenting hängt u.a. auch davon ab, wie gleichförmig sich der Stent an die Gefäßwand anlegen kann. Denn umso gleichförmiger die Gefäßwand abgestützt wird, um so wahrscheinlicher ist es, daß es im Bereich der Stents nicht erneut zu Gefäßverengungen kommt. Dabei bewirkt eine gleichmäßige Stent-Struktur eine relativ glatte Gefäßinnenoberfläche und bei einer relativ glatten Gefäßinnenoberfläche können sich Blutpartikel nur schwer an diese anlagern. Darüber hinaus werden auch Wucherungen der Intima in das Gefäßinnere stärker durch eine gleichmäßige Stentstruktur, die die Gefäßinnenoberfläche relativ geschlossen abgedeckt, verhindert.

Derartige Stents mit einer sogenannten geschlossenen Struktur sind ebenfalls aus dem Stand der Technik bekannt. Beispielhaft sei hier einer der bekanntesten derartiger Stents herausgegriffen, der sogenannte Wallstent. Dieser ist beispielsweise aus dem US 4,655,771 bekannt. Dieser eine geschlossene Struktur aufweisende Stent ist aus mehreren gleichmäßig maschenartig gewirkten, in Längsachse des Stents spiralförmig verlaufenden Drähten gebildet.

Der Vorteil der geschlossenen Struktur derartiger Stents wird jedoch durch den Nachteil erkauft, daß diese Stents eine relative longitudinale Steifheit während des Einsetzens aufweisen. Diese Stents erlauben es daher nicht in optimaler Weise, den Stent beim Einführen in Richtung auf die zu behandelnde Stenose durch möglicherweise sehr stark gekrümmte Gefäßabschnitte der Herzkranzarterien zu führen. Um diese Nachteile einer geschlossenen Struktur zu vermeiden, wurden nunmehr Stents entwickelt, die einen sogenannten modularen Aufbau aufweisen. Bei diesen modularartig aufgebauten Stents sind einzelne, mit einer geschlossenen Struktur versehene Abschnitte, durch flexible Verbindungen miteinander verbunden. Derartige Stents sind beispielsweise aus dem US-Patent 5,104,404 bekannt.

Nachteilig bei diesen modularen bzw. segmentierten Stents ist es jedoch, daß sich die in Einführrichtung des Stents vorne liegenden Vorderkanten jedes einzelnen Moduls bzw. Segmentes in der Gefäßinnenwand verhaken können. Auf diese Weise kann es zu erheblichen Komplikationen bei dem Einführen eines Stents kommen. Dies ist insbesondere insoweit besonders problematisch, da die modularen Stents - wie oben bereits ausgeführt - vor allem dann zum Einsatz gelangen, wenn erhebliche Krümmungen auf dem Weg zu der behandelnden Stelle zu überwinden sind. Denn in einer solchen Krümmung verhakt sich eine solche Vorderkante eines Segmentes besonders leicht an der in der Krümmung außenliegenden lnnenoberfläche des Gefäßes, durch die der Stent hindurchgeführt wird.

Aufgabe der Erfindung ist es daher, die vorgenannten Nachteile zu vermeiden und einen Stent der eingangs genannten Art zur Verfügung zu stellen, welcher sowohl an dem Ort der zu behandelnden Stenose eine geschlossene Abdeckung der Gefäßinnenoberfläche ermöglicht, während er gleichzeitig ausreichend flexibel ist, um an diesem Ort verschoben werden zu können.

Diese Aufgabe wird bei der Erfindung mit einem Stent der eingangs genannten Art dadurch gelöst, daß die in dem Stent vorgesehenen Ausnehmungen im wesentlichen T-förmig ausgebildet sind. T-förmige Ausnehmungen sind in DE-A-19 722 857 offenbart. Sie sind mit dem Querbalken in Umfangsrichtung orientiert.

Die Vorteile der Erfindung liegen insbesondere darin, daß durch die T-förmigen Ausnehmungen in der rohrförmigen Struktur des Stents sowohl eine insgesamt geschlossene Struktur realisiert wird, so daß eine gute Abdeckung der Läsion erreicht wird, während gleichzeitig aufgrund der T-förmigen Ausnehmungen eine gegenüber den aus dem Stand der Technik bekannten geschlossenen Stentstrukturen erhöhte Flexibilität in longitudinaler Richtung des Stents erreicht wird.

Die Erfindung schließt die Erkenntnis ein, daß durch die erfindungsgemäß T-förmig ausgebildeten Ausnehmungen diese Ausnehmungen zwei Abschnitte aufweisen, die im wesentlichen senkrecht aufeinander stehen. Es handelt sich dabei zum einen um den den Querbalken des T bildenden Abschnitt der Ausnehmung und um den den Standbalken des T bildenden Abschnitt der Ausnehmung. Dabei läuft der Querbalken des T in longitudinaler Richtung des rohrförmigen Abschnittes des Stents, während der Standbalken des T bevorzugt in Umfangsrichtung des rohrförmigen Abschnittes des erfindungsgemäßen Stents verläuft. Auf diese Weise wird sichergestellt, daß in beiden vorgenannten Richtungen eine Flexibilität besteht, die dem erfindungsgemäßen Stent insgesamt ein Maß an Flexibilität verleiht, wie es beispielsweise beim Hindurchschieben des Stents durch Krümmungen in Herzkranzgefäßen erforderlich ist.

Bei einer bevorzugten Ausbildung der vorliegenden Erfindung weist der rohrförmige Abschnitt eine Mantelfläche auf, wobei die Ausnehmungen in dem rohrförmigen Abschnitt von Stegen begrenzt sind, welche Stege aus dem verbleibenden Material einer die Mantelfläche bildenden Rohrwandung des rohrförmigen Abschnittes gebildet sind, von welcher Rohrwandung das Material im Bereich der Ausnehmungen entfernt wurde. Diese Ausführungsform zeichnet sich dadurch aus, daß die Stege die Ausnehmungen in Richtung der Längsachse des rohrförmigen Abschnittes im wesentlichen in der Form eines S begrenzen. Diese Ausführungsform zeichnet sich vorteilhafterweise dadurch aus, daß durch die S-förmigen Begrenzungsstege für die Ausnehmungen eine erhöhte Flexibilität des Stents erreicht wird, da die S-Form von Haus aus flexibel ist. Darüber hinaus ist es bei dieser Ausführungsform bevorzugt, wenn die S-förmigen Stege so angeordnet sind, daß das jeweils gebildete S in Umfangsrichtung steht. Denn auf diese Weise ist es möglich, daß die jeweiligen Bäuche des S bei Einschieben des Stents in gekrümmte Herzkranzgefäße sich in die Nachbarbeugung bzw. den Nachbarbauch des gleichen S, oder des umfangsseitig benachbarten S der Stege hineinverschieben, um so die Flexibilität des Stents zu gewährleisten. Ein weiterer Vorteil dieser Ausführungsform ist es, daß beim Crimpen des Stents ein kleineres Crimp-Profil entsteht. Dieses kleinere Crimp-Profil wird bei dieser Ausführungsform durch die S-förmigen Stege ermöglicht, ohne daß sich die Stege gegenseitig berühren. Durch den kleineren Durchmesser des somit erreichbaren Crimp-Pprofils wird der Zugang zu Stenosen und die Erreichbarkeit entlegener Stenosen mit Hilfe eines derartigen Stents erleichtert.

Entsprechend der Erfindung sind die Ausnehmungen bezüglich einer sie schneidenden Umfangslinie spiegelsymmetrisch ausgebildet. Auf dieses Weise wird sichergestellt, daß der Stent bei seiner Ausdehnung gleichförmig expandiert, ohne daß es zu einem Schiefziehen der Stentstruktur bei der Expansion kommt.

Eine weitere Fortbildung der Erfindung zeichnet sich dadurch aus, daß die Stege derart die Ausnehmungen begrenzen, daß die die Standbalken des T bildenden Teile der T-förmigen Ausnehmungen im wesentlichen in Umfangsrichtung der rohrförmigen Abschnitte verlaufen. Dabei ist es weiter bevorzugt, wenn die Stege derart die Ausnehmungen begrenzen, daß die die Querbalken der T-förmigen Ausnehmungen bildenden Teile der Ausnehmungen im wesentlichen in Längsrichtung der rohrförmigen Abschnitte der Stents verlaufen. Durch diese regelmäßige Anordnung der Stege wird eine optimal geschlossene Struktur mit ihren eingangs genannten Vorteilen erzeugt. Gleichzeitig wird jedoch durch die auf diese Weise T-förmig ausgebildeten Ausnehmungen erreicht, daß die den Querbalken des T bildenden Teile der Ausnehmungen optimal lateral beweglich sind, wie es beispielsweise beim Einführen des Stents in starke Krümmungen bei Herzkranzgefäßen von erheblichem Vorteil ist.

Bei einer weiter bevorzugten Ausführungsform der vorliegenden Erfindung sind die Abschnitte der Stege, die die Enden der S-förmigen, die Ausnehmungen in Längsrichtung des rohrförmigen Abschnittes begrenzenden Stege bilden, jeweils mit den bezüglich der durch sie umfaßten, T-förmigen Ausnehmung spiegelsymmetrisch gegenüberliegenden, die entsprechenden Enden bildenden Abschnitte über Verbindungsmittel verbunden. Dabei ist es bevorzugt, wenn diese Verbindungsmittel im wesentlichen in Längsrichtung des rohrförmigen Abschnittes des erfindungsgemäßen Stents verlaufende Stege sind. Diese Stege können weiter bevorzugt in lateraler Richtung leicht gebogen ausgebildet sein, um die Flexibilität des Stents auch in diesem Bereich der Verbindungsmittel zu erhöhen. Insgesamt läßt sich jedoch durch diese Ausbildung der Erfindung eine optimale Einleitung der verschiedenen, bei Flexbewegungen des rohrförmigen Abschnittes auftretenden Kräfte in die Gesamtstruktur des Stents erzielen.

In einer weiter bevorzugten Ausführungsform der vorliegenden Erfindung sind die S-förmigen Stege an mindestens einem der Bäuche des S eingestülpt ausgebildet. Diese Einstülpungen, die weiter bevorzugt um mindestens 50% der Ausbauchung des entsprechenden S in diese Ausbauchung eingestülpt sind, läßt sich besonders vorteilhaft eine axiale Verkürzung des Stents bei radialer Ausdehnung des Stents erfolgreich vermeiden. Denn durch diese in die bauchförmigen Bereiche der S-förmigen Stege eingearbeiteten Einstülpungen wird radiales Ausdehungspotential für den Stent zur Verfügung gestellt, ohne daß dies auf Kosten der Länge des Stents geht. Darüber hinaus sorgen auch diese Einstülpungen für eine weiter erhöhte Flexibilität des Stents, da diese Ausstülpungen die laterale Beweglichkeit der S-förmigen Stege weiter erhöhen.

Diese auch als Doppelkurve zu bezeichnenden Gebilde aus S-förmigen Stegen und Einstülpungen in den Bäuchen der S-förmigen Stege dienen also in ganz besonderer Weise zur Verwirklichung der erfindungsgemäßen Aufgabe.

Bei einer weiteren Fortbildung des erfindungsgemäßen Stents ist der den Querbalken des T bildende Teil der Ausnehmung breiter als der den Standbalken des T bildende Teil. Mit Hilfe dieser Ausführungsform ist es vorteilhaft möglich, den Stent auch im Bereich von Gefäßverzweigungen bei dem Stenting von Herzkranzgefäßen einzusetzen. Es ist bei dieser Ausführungsform ein leichtes, Fenster in ein derartiges Design einzufügen. Denn aufgrund der geschlossenen, homogenen und regelmäßigen Struktur wirkt sich eine derartige Fensterung eines bestimmten Bereiches des rohrförmigen Abschnittes des erfindungsgemäßen Stents nicht nachteilig auf die Gesamtstrukturfestigkeit aus. Dies ergibt sich unter anderem auch durch die Verschachtelung der T-förmigen Ausnehmungen. Denn durch diese Verschachtelung ergibt sich wiederum eine vor allem gleichmäßige Abdeckung der Mantelfläche des Stents im expandierten Zustand, so daß im expandierten Zustand des Stents keine größeren zusammenhängenden freien Flächen in der Mantelfläche offenstehen. Somit erweist sich die erfindungsgemäße, geschlossene und homogene Stentstruktur nicht nur als flexibler gegenüber dem bekannten Stand der Technik derartiger geschlossener Strukturen; vielmehr ist es mit Hilfe dieser Struktur auch ohne weiteres möglich, Fenster für Gefäßverzweigung in dem Stent vorzusehen. Denn derartige Fensterungen sind im Stand der Technik bei den bisher bekannten geschlossenen Strukturen nicht ohne weiteres möglich, da diese zu eng strukturiert sind, d.h. keine ausreichenden Ansetzmöglichkeiten zum Einfügen eines derartigen Fensters bieten.

Ein weiterer Vorteil der bereits oben erwähnten Verschachtelung wird bei der Betrachtung des Querschnitts des expandierten Stents, also bei einer Betrachtung in axialer Richtung, deutlich. Denn die verformbaren T-förmigen Elemente des erfindungsgemäßen Stents werden durch die obenen genannten Einstülpungen in die Bäuche der S-förmigen Stege weiter unterteilt. Dies hat zur Folge, daß der Stent insgesamt und insbesondere in Umfangsrichtung aus verhältnismäßig kurzen Stegen aufgebaut ist. Die die T-förmigen Ausnehmungen begrenzenden Stege weisen daher nur relativ kurze gerade Stegabschnitte auf. Da jedoch bei dem Expandieren des Stents die Verformungen der Stege vor allem im Bereich der Radien, d.h. der Biegungen in den Stegen auftreten, liegt bei dem erfindungsgemäßen Stent eine Annäherung des expandierten Stent-Querschnittes an die Kreisform in besonderer Weise vor. Dies verbessert die Anpaßbarkeit des erfindungsgemäßen Stents an das umliegende Gefäß gegenüber dem bekannten Stents aus dem Stand der Technik. Denn die bekannten Stents aus dem Stand der Technik weisen nur relativ lange Stegsegmente auf, die sich bei dem Expandieren des Stents bezüglich des Stentsquerschnittes nur unzureichend an die Kreisform annähern. Im Stand der Technik ragen daher nachteiligerweise einzelne Stegabschnitte beim expandierten Stent in den Innenraum des Gefäßes hinein und behindern somit nachteiligerweise den freien Durchfluß von Blut.

Eine besonders bevorzugte Ausführungsform der Erfindung zeichnet sich dadurch aus, daß alle Ausnehmungen gleich groß sind. Der Stent ist demnach bevorzugt aus sich gleichmäßig wiederholenden, immer gleichen Strukturelementen oder sogenannten Einheitszellen aufgebaut.

Weitere bevorzugte Ausführungsformen der Erfindung sind in den Unteransprüchen angegeben.

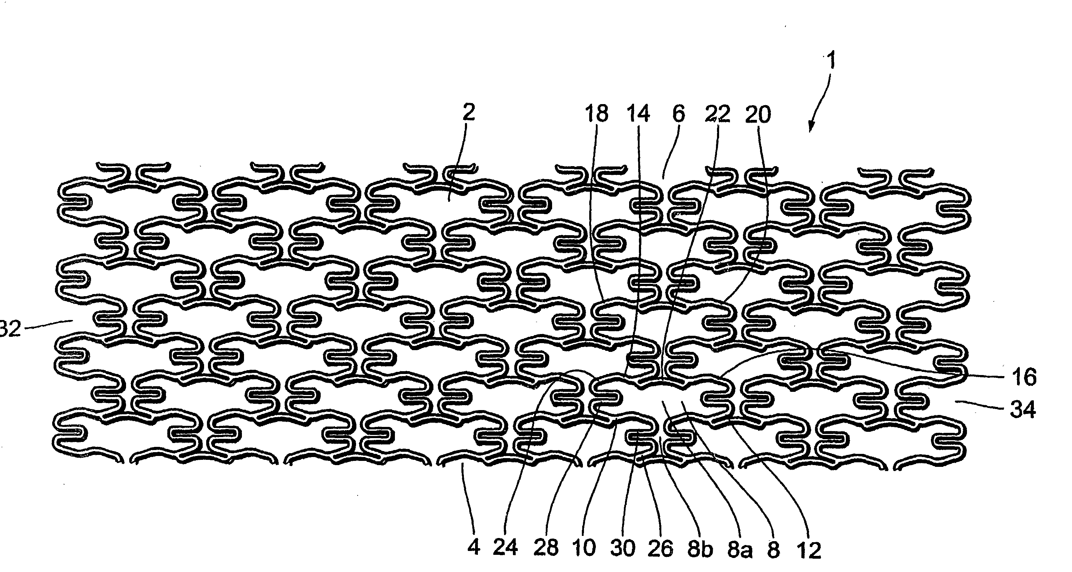

Ein Ausführungsbeispiel der vorliegenden Erfindung wird nun anhand der einzigen Figur beschrieben.

Die Figur zeigt einen erfindungsgemäßen Stent 1. Der Stent 1 ist in der Figur als Abwicklung der Mantelfläche 2 des Stents 1 dargestellt. Im funktionsfähigen Zustand des Stents 1 ist die Mantelfläche 2 mit ihrer in der Figur unten dargestellten Seite 4 und in der Figur oben dargestellten Seite 6 aneinandergefügt, so daß sich ein die Mantelfläche 2 aufweisender rohrförmiger Abschnitt ergibt.

Die Mantelfläche 2 weist erste, T-förmige Ausnehmungen 8 auf. Alle T-förmigen Ausnehmungen 8 sind identisch geformt und gleich groß. Die T-förmigen Ausnehmungen 8 sind auf der Mantelfläche ineinander verschachtelt angeordnet. Die T-förmigen Ausnehmungen weisen einen breiten, den Querbalken des T bildenden, in der Figur jeweils oben dargestellten Oberteil 8a, und einen den Standbalken des T, in der Figur unten dargestellten Untereil 8b auf. Der Oberteil 8a und der Unterteil 8b bilden somit die T-förmige Ausnehmungen 8.

Die T-förmigen Ausnehmungen 8 sind in der Figur jeweils auf der linken Seite von einem S-förmigen Steg 10 begrenzt. Spiegelsymmetrisch zu einer die jeweiligen Ausnehmungen 8 jeweils in der Mitte schneidenden Umfangslinie, die in der Figur jeweils von oben nach unten senkrecht verläuft, ist dem jeweiligen S-förmigen Steg 10 ein spiegelverkehrt-S-förmiger Steg 12 angeordnet.

An den oberen Enden 14 bzw. 16 der Stege 10 bzw. 12 sind diese in Umfangsrichtung der mantelflächig 2 gesehen mit jeweils identischen Stegen 18 bzw. 20 verbunden. Zusammen bilden die S-förmigen Stege 10 und 18 bzw. 12 und 20 in Umfangsrichtung laufende Rechteckkurven.

Darüber hinaus sind die Stege 10 bzw. 12, die vorstehend und auch im folgenden nur beispielhaft aus der Gesamtstruktur in der Figur herausgegriffen sind, mit ihren Enden 14 bzw. 16 auch miteinander über einen als erfindungsgemäßes Verbindungsmittel dienenden, in der Figur leicht nach oben bogenförmig ausgebildeten Verbindungssteg 22 verbunden. Der Verbindungssteg 22 läuft dabei im wesentlichen in longitudinaler Richtung des Stents 1. Jede der den beispielhaft herausgegriffenen Enden 14 und 16 entsprechenden Enden der dargestellten Stege 10 bzw. 12 sind miteinander über derartige Verbindungsstege 22 verbunden. Auch sind alle Verbindungsstege 22 exakt identisch. Die Verbindungsstege 22 greifen an längenneutralen Punkten der Enden 14 und 16 der Stege 10 bzw. 12 an. Auf diese Weise wird die axiale Verkürzung des Stents 1 beim Expandieren des Stents 1 minimiert.

Die flächenmäßige Ausdehnung des den Querbalken der T-förmigen Ausnehmungen 8 bildenden Teils 8a ist etwa vier bis fünf mal so groß wie die den Standbalken der Ausnehmungen 8 bildenden Teils 8b. Insbesondere die in Längsausdehnung der jeweiligen Teile 8a und 8b gemessene Breite dieser Ausnehmungsteile unterschiedlich, so daß die Breite des den Querbalken bildenden Teils 8a etwa doppelt bis dreimal so groß ist wie die Breite des den Standbalken bildenden Teils 8b der Ausnehmung 8.

Weiterhin weisen die die T-förmigen Ausnehmungen 8 begrenzenden, S-förmigen Stege 10 bzw. 12 die an sich die S-Form der Stege erzeugenden Ausbauchungen 24 und 26 auf. In diese die eigentliche S-Form der Stege 10 bzw. 12 bildenden Ausbauchungen 24 und 26 sind entgegen der Ausbauchungsrichtung eingearbeitete Einstülpungen 28 bzw. 30 vorgesehen. Diese Einstülpungen ragen im Bereich der Oberteile 8a in die Ausnehmung 8 hinein, während sie die Ausnehmung 8 im Bereich der Unterteile 8b erweitern. Aufgrund der verschachtelten Struktur stellen die Erweiterungen der Unterteile 8b jedoch gleichzeitig die Einschnürungen im Bereich der Oberteile 8a dar.

Aufgrund der S-förmigen Umrandungen der Ausnehmungen 8 kann die gesamte Mantelfläche der Stentstruktur an den Rändern 32 (in der Figur links) und 34 (in der Figur rechts) ebenfalls geschlossen ausgebildet werden, so daß sich keine spitzen Enden ergeben, die möglicherweise beim Einführen des Stents Körperöffnungen einhaken könnten.

## Patentansprüche

1. Stent, insbesondere Koronarstent (1), mit einem rohrförmigen Abschnitt, der Ausnehmungen (8) aufweist, **dadurch gekennzeichnet, dass** die Ausnehmungen (8) im wesentlichen T-förmig und zu einer entlang des Umfangs durch sie hindurchlaufenden Linie spiegelsymmetrisch ausgebildet sind.

2. Stent nach Anspruch 1, wobei der rohrförmige Abschnitt eine Mantelfläche (2) aufweist, wobei die Ausnehmungen (8) von Stegen (10, 12, 18, 20) begrenzt sind, welche Stege (10, 12, 18, 20) aus dem restlichen Material einer die Mantelfläche (2) bildenden Rohrwandung des rohrförmigen Abschnittes gebildet sind, von welcher Rohrwandung das Material im Bereich der Ausnehmungen (8) so entfernt wurde, da es zu einer Verschachtelung der T-förmigen Ausnehmungen kommt, wobei die Stege (10, 12, 18, 20) die Ausnehmungen (8) in Richtung der Längsachse des rohrförmigen Abschnittes im wesentlichen S-förmig begrenzen.

3. Stent nach Anspruch 2, wobei die Stege (10, 12, 18, 20) derart angeordnet sind, dass die die Standbalken der T-förmigen Ausnehmungen (8) bildenden Teile (8b) der Ausnehmungen (8) im wesentlichen in Umfangsrichtung der rohrförmigen Abschnitte verlaufen.

4. Stent nach einem der Ansprüche 2 oder 3, wobei die Stege (10, 12, 18, 20) derart angeordnet sind, dass die die Querbalken der T-förmigenAusnehmungen (8) bildenden Teile (8a) der Ausnehmungen (8) im wesentlichen in Längsrichtung der rohrförmigen Abschnitte verlaufen.

5. Stent nach einem der Ansprüche 2 bis 4, wobei die Abschnitte der Stege (10, 12, 18, 20), die die Enden (14, 16) der S-förmigendie Ausnehmungen (8) in Längsrichtung des rohrförmigen Abschnittes begrenzenden Stege (10, 12, 18, 20) bilden, jeweils mit den bezüglich der durch die Stege (10, 12, 18, 20) umfassten T-förmigen Ausnehmung (8) spiegelsymmetrisch gegenüberliegenden, die entsprechenden Enden (14, 16) bildenden Abschnitten über Verbindungsmittel (22) verbunden sind.

6. Stent nach Anspruch 5, wobei die Verbindungsmittel im wesentlichen in Längsrichtung des rohrförmigen Abschnittes verlaufende Stege (22) sind.

7. Stent nach einem der Ansprüche 2 bis 6, wobei die S-förmigen Stege (10, 12, 18, 20) an mindestens einem der Bäuche (24, 26) des S eine Einstülpung (28, 30) aufweisen.

8. Stent nach Anspruch 7, wobei die Einstülpung (28, 30) entgegen der Wölbung des entsprechenden Bauches (24, 26) des S-förmigen Steges (10, 12, 18, 20) ausgebildet ist.

9. Stent nach einem der vorstehenden Ansprüche, wobei der den Querbalken des T bildende Teil (8a) der Ausnehmung (8) breiter ist als der den Standbalken des T bildende Teil (8b) der Ausnehmung (8).

10. Stent nach einem der vorstehenden Ansprüche, wobei alle Ausnehmungen (8) gleich groß sind.

## Claims

1. Stent, in particular a coronary stent (1), having a tubular portion comprising openings (8), **characterised in that** the openings are substantially T-shaped and are constructed mirror-inverted with respect to a line extending through the openings in the peripheral direction.

2. Stent according to claim 1, wherein the tubular portion has a peripheral surface (2) , wherein the openings (8) are defined by webs (10, 12, 18, 20), which webs (10, 12, 18, 20) are formed from the remaining material of a tubular wall, forming the peripheral surface (2), of the tubular portion, from which tubular wall the material has been removed in the region of the openings (8) such that there is interlocking of the T-shaped openings, and wherein the webs (10, 12, 18, 2) define in a substantially S-shaped manner the openings (8) in the direction of the longitudinal axis of the tubular portion.

3. Stent according to claim 2, wherein the webs (10, 12, 18, 20) are arranged in such a way that the portions (8b) of the openings (8) forming the upright bars of the T-shaped openings (8) extend substantially in the peripheral direction of the tubular portions.

4. Stent according to either of claims 2 or 3, wherein the webs (10, 12, 18, 20) are arranged in such a way that the portions (8a) of the openings (8) forming the crossbars of the T-shaped openings (8) extend substantially in the longitudinal direction of the tubular portions.

5. Stent according to any of claims 2 to 4, wherein the portions of the webs (10, 12, 18, 20) forming the ends (14, 16) of the S-shaped webs (10, 12, 18, 20) defining the openings (8) in the longitudinal direction of the tubular portion are respectively connected via connecting means (22) to the portions forming the corresponding ends (14, 16) and disposed in an opposing and mirror-inverted manner with respect to the T-shaped opening (8) incorporated by the webs (10, 12, 18, 20).

6. Stent according to claim 5, wherein the connecting means are webs (22) extending substantially in the longitudinal direction of the tubular portion.

7. Stent according to any of claims 2 to 6, wherein the S-shaped webs (10, 12, 18, 20) have an inwardly inverted portion (28, 30) on at least one of the bulges (24, 26) of the S.

8. Stent according to claim 7, wherein the inwardly inverted portion (28, 30) is constructed opposite the curve of the corresponding bulge (24, 26) of the S-shaped web (10, 12, 18, 20).

9. Stent according to any of the preceding claims, wherein the portion (8a) of the opening (8) forming the crossbar of the T is wider than the portion (8b) of the opening (8) forming the upright bar of the T.

10. Stent according to any of the preceding claims, wherein all openings (8) are of equal size.

## Revendications

1. Extenseur, notamment extenseur coronarien (1), comportant une section de forme tubulaire, qui possède des évidements (8), **caractérisé en ce que** les évidements (8) sont réalisés essentiellement en forme de T et sont symétriques par rapport à une ligne qui s'étend le long de la circonférence et traverse ces évidements.

2. Extenseur selon la revendication 1, dans lequel la section de forme tubulaire comporte une surface enveloppe (2), et dans lequel les évidements (8) sont limités par des barrettes (10, 12, 18, 20), lesquelles barrettes (10, 12, 18, 20) sont formées par le matériau résiduel d'une paroi tubulaire, de la section tubulaire, qui forme la surface enveloppe (2), paroi tubulaire à partir de laquelle le matériau dans la zone des évidements (8) a été retiré, et dans lequel, étant donné qu'il se produit une imbrication des évidements en forme de T, les barrettes (10, 12, 18, 20) délimitent essentiellement en forme de S les évidements (8) dans la direction de l'axe longitudinal de la section de forme tubulaire.

3. Extenseur selon la revendication 2, dans lequel les barrettes (10, 12, 18, 20) sont disposées de telle sorte que les parties (8b) des évidements (8), qui forment les branches verticales des évidements (8) en forme de T, s'étendent essentiellement dans la direction circonférentielle des sections de forme tubulaire.

4. Extenseur selon l'une des revendications 2 ou 3, dans lequel les barrettes (10, 12, 18, 20) sont disposées de telle sorte que les parties (8a) des évidements (8), qui forment les branches transversales des évidements en forme de T (8) s'étendent essentiellement dans une direction longitudinale des sections de forme tubulaire.

5. Extenseur selon l'une des revendications 2 à 4, dans lequel les sections des barrettes (10, 12, 18, 20), qui forment les extrémités (14, 16) des barrettes en forme de S (10, 12, 18, 20), qui délimitent les évidements (8) dans la direction longitudinale de la section de forme tubulaire, sont reliées respectivement aux sections qui sont opposées symétriquement par rapport à l'évidement en forme de T (8) entouré par les barrettes (10, 12, 18, 20) et forment les extrémités correspondantes (14, 16), par des moyens de liaison.

6. Extenseur selon la revendication 5, dans lequel les moyens de liaison sont des barrettes (22) qui s'étendent essentiellement dans la direction longitudinale de la section de forme tubulaire.

7. Extenseur selon l'une des revendications 2 à 6, dans lequel les barrettes en forme de S (10, 12, 18, 20) possèdent un renfoncement (28, 30) sur au moins l'une des parties renflées (24, 26) du S.

8. Extenseur selon la revendication 7, dans lequel l'enfoncement (28, 30) est agencé à l'encontre du bombement de la partie renflée correspondante (24, 26) de la barrette en forme de S (10, 12, 18, 20).

9. Extenseur selon l'une des revendications précédentes, dans lequel la partie (8a), qui forme la barre transversale du T, de l'évidement (8) est plus large que la partie (8b), qui forme la barre verticale de la partie du T, de l'évidement (8).

10. Extenseur selon l'une des revendications précédentes, dans lequel tous les évidements (8) ont une même taille.
